# EUROPÄISCHE PATENTSCHRIFT

(11) **EP 0 410 010 B1**
(45) Veröffentlichungstag und Bekanntmachung des Hinweises auf die Patenterteilung: **27.10.1993**
(21) Anmeldenummer: 89113484.3
(22) Anmeldetag: 22.07.1989
(51) Int. Cl.: A61L 27/00

(54) **Osteotropes Implantatmaterial**
Osteotropic implant material
Matériau d'implantation ostéotropique

(43) Veröffentlichungstag der Anmeldung: 30.01.1991
(73) Patentinhaber: Osborn, Johannes Friedrich, Prof. Dr., 53115 Bonn (DE)
(72) Erfinder: Osborn, Johannes Friedrich, Prof. Dr., 53115 Bonn (DE)
(74) Vertreter: Müller-Wolff, Thomas, Dipl.-Ing.

(56) Entgegenhaltungen:
- EP-A- 0 006 544
- EP-A- 0 202 908
- EP-A- 0 232 791
- FR-A- 2 283 104
- FR-A- 2 548 540
- PHILIPS TECHN. REV., Band 37, Nrs. 9/10, 1977, Seiten 234-236, Philips BV, Eindhoven, NL; J.G.J. PEELEN et al.: "Sintered hydroxylapatite as a bioceramic"

## Beschreibung

Die Erfindung betrifft ein osteotropes Implantatmaterial enthaltend austauschbare, biokompatible Ionen, wobei das Implantatmaterial eine Mikroporosität mit Porendurchmessern < 5 m von 3-20 Vol% und eine Makroporosität mit Porenweiten > 100 m von 10-60 Vol% aufweist.

In der Fachliteratur hat sich der Begriff der "Osteotropie" für die Fähigkeit eines Materials eingebürgert, im Kontakt mit Knochengewebe die Bildung von neuem Knochen hervorzurufen und zu beschleunigen (s. Tagungsbericht des 3. Weltkongresses für Biomaterialien vom 21. bis 25. April 1988 in Kyoto, Japan).

Die bisher für Implantatzwecke verwendeten Materialien aus Biokeramik wiesen eine Makroporosität mit einer Porenweite von 200-800 m bei einer spezifischen Oberfläche von 0,07m²/g auf. Bei Untersuchungen an diesen Materialien wurde festgestellt, daß der zeitliche Verlauf der Knochenneubildung und die Menge des neugebildeten Knochens unbefriedigend waren.

Weitere Untersuchungen zeigten, daß makroporöses Material, z. B. Kalziumphosphatwerkstoff, eine höhere Osteotropie aufwies als porenfreies, dichtes Kalziumphosphat. Den größten Osteotropieeffekt zeigten Kalziumphosphatwerkstoffe mit einer Mikro/Makroporösität, wobei deren Verteilung herstellungsbedingt gesteuert werden kann.

Nach Philips, Techn. Rev. 37, Seite 235, linke Spalte, Mitte, kann die Mikroporosität durch geeignete Sinterbedingung und die Makroporosität durch die stoffliche Zusammensetzung in die Trocknungsrate des Implantatmaterials gesteuert werden. So ist beispielsweise eine Hydroxylapatitkeramik bekannt, die eine Makroporosität von 20 Vol% bei einer Porenweite von 50-250 µm und eine Mikroporosität von 0,5-1,5 µm bei einem Volumenanteil von 20 Vol% aufweist. Die Verwendung als Implantat ist jedoch unbefriedigend, da die Knochenneubildung sehr unterschiedlich verläuft und die Aktivierung der knochennbildenden Zellen zu langsam erfolgt.

Aufgabe der vorliegenden Erfindung ist es daher, ein Implantat mit gegenüber den bisherigen Materialien verbesserten osteotropen Eigenschaften, insbesondere einer hohen Knochenneubildung und einem günstigen zeitlichen Verlauf der Knochenbildung herzustellen. Die Aufgabe wird durch die in den Patentansprüchen angegebenen Merkmale gelöst.

Es hat sich gezeigt, daß bestimmte Implantatwerkstoffe in wäßriger Lösung eine ruhende Schicht aufbauen, die ein elektrisches Potential (gemessen zwischen ruhender Schicht und umgebender Lösung) aufweist, das im Bereich des natürlichen Potentials liegt, gemessen in gleicher Umgebung am mazerierten, d.h. von allen organischen Bestandteilen befreiten Knochen. Nur solche Materialien, auf denen eine ruhende Schicht existiert, zeigen eine wesentlich frühere Aktivierung der zur Knochenneubildung fähigen Zellen und vermögen eine größere Menge an neuem Knochen hervorzurufen als vergleichbare Implantate ohne ruhende Schicht.

Erfindungsgemäß wird das Potential der ruhenden Schicht als Meßgröße zur Bestimmung der Osteotropie-Kapazität herangezogen. Es ist demnach eine äquivalante Beziehung zwischen dem Potential der ruhenden Schicht und der Osteotropie-Kapazität zugrundegelegt.

Im folgenden wird die Erfindung anhand eines Beispieles näher erläutert.

Die
- Figur 1: zeigt ein Prinzipbild von einem Implantat mit ruhender Schicht.
- Figur 2: zeigt eine Meßanordnung zur Messung des elektrischen Potentials.
- Figur 3: zeigt eine Gegenüberstellung der an verschiedenen Implantatmaterialen vorliegenden Potentialverhältnisse.

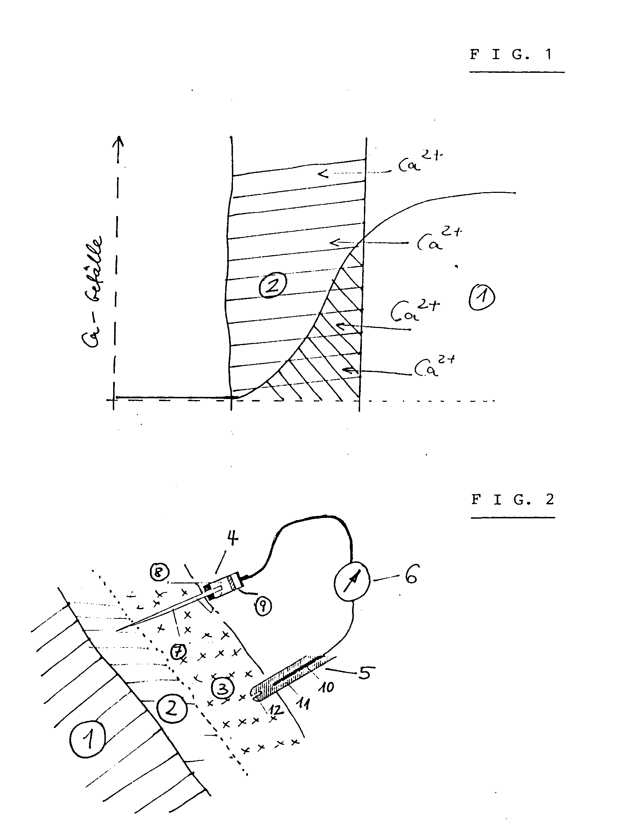

In Fig. 1 ist die Grenzfläche an einer Hydroxylapatitkeramik in wäßriger Umgebung schematisch dargestellt. Dabei besteht der Festkörper 1 aus polarisierten und unpolarisierten Calzium- und Phosphationen. An der Grenzschicht zur Flüssigkeit 3 hin bildet sich eine ruhende Schicht 2 aus polarisierten Calzium- und Phosphationen aus. In die Flüssigkeit sind gemäß Fig. 2 die Elektroden 4,5 eingetaucht. Die Elektrode 4 besteht aus einer Glasmikropipette 7 mit innerhalb des Elektrodenhalters 8 angeordnetem Filament 9 aus Silber/Silberchlorid. Der Halter 8 und die Pipette 7 sind mit 50mM KCl gefüllt. Die Elektrode 5 besteht aus einer Referenzelektrode mit innenliegendem Silber/Silber-Chloriddraht 10, der von einer 3M KCL-Lösung 11 umgeben wird, die in einem Keramikdiaphragma 12 eingeschlossen ist. Mit Voltmeter 6 kann das Potential der ruhenden Schicht gemessen werden.

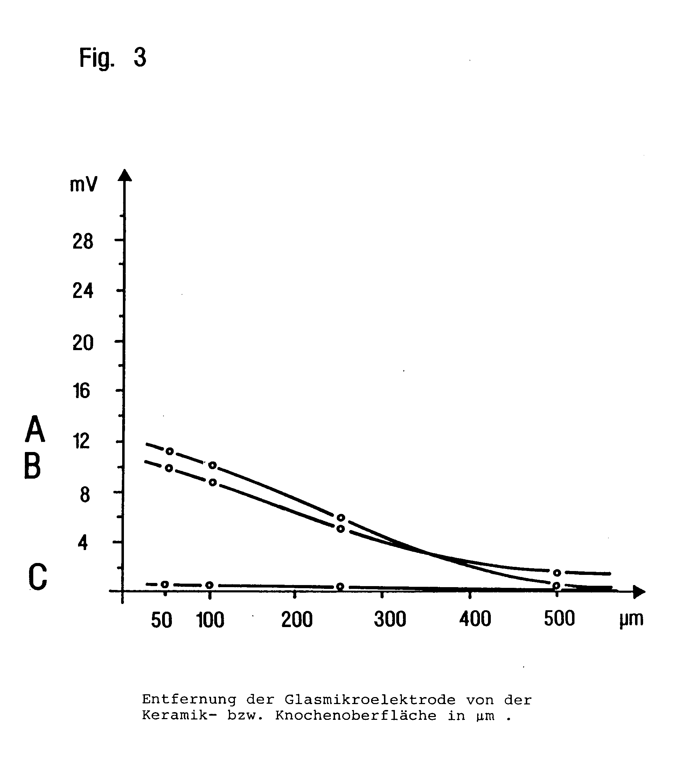

In Fig. 3 sind die mit der Elektrode gemessenen Spitzenpotentiale in Abhängigkeit von Objektabstand für verschiedene keramische Materialien dargestellt. Es zeigt sich, daß das Material A annähernd das gleiche Potentialverhalten wie das natürliche Knochenmaterial aufweist.

Im Rahmen von Vergleichsversuchen wurde gefunden, daß eine ruhende Schicht mit konstanter Dicke und reproduzierbaren Potentialwerten sich immer dann ausbildet, sofern alle anhaftenden Teilchen von der Rohkeramik entfernt sind. Dies geschieht erfindungsgemäß durch ein Ultraschallreinigungsverfahren, das die Mikro- und Makroporen auch von feinsten Staubteilchen befreit.

Untersuchte Materialien gemäß Fig. 3:
A: mazerierter Knochen
B: Mikro-makroporöse Hydroxylapatit-Keramik (HAK), ultraschallgereinigt
C: dichte Al₂O₃-Keramik

## Patentansprüche

1. Osteotropes Implantatmaterial enthaltend austauschbare, biokompatible Ionen, wobei das Implantatmaterial eine Mikroporosität mit Porendurchmessern < 5 µm von 3-20 Vol% und eine Makroporosität mit Porenweiten > 100µm von 10-60 Vol% aufweist,
dadurch gekennzeichnet,
daß Implantatmaterial in wässrigen Lösungen eine ruhende Schicht 2 (Fig.1) aus polarisierten Calzium- und Phosphationen aufweist.

2. Osteotropes Implantatmaterial nach Anspruch 1,
dadurch gekennzeichnet,
daß die Dicke der ruhenden Schicht 5-500µm beträgt.

3. Osteotropes Implantatmaterial nach einem der vorhergehenden Ansprüche dadurch gekennzeichnet, daß zwischen der ruhenden Schicht des Implantatmaterials und der 1%-igen Kochsalzlösung ein Potential von 10-13 mV herrscht, gemessen mit einer Glasmikroelektrode.

4. Osteotropes Implantatmaterial nach einem der vorhergehenden Anspüche dadurch gekennzeichnet, daß sich in der ruhenden Schicht ausgetauschte, biokompatible Ionen befinden.

5. Osteotropes Implantatmaterial nach einem der vorhergehenden Ansprüche dadurch gekennzeichnet, daß das Implantatmaterial aus Hydroxylapatitkeramik besteht und die ruhende Schicht Calcium-Ionen aufweist.

6. Verfahren zur Herstellung eines osteotropen Implantatmaterials nach einem der vorhergehenden Ansprüche dadurch gekennzeichnet, daß das Implantatmaterial in eine verdünnte Kochsalzlösung getaucht wird, nach Ausbildung einer ruhenden Schicht eine Glasmikroelektrode in die Schicht eingeführt wird und zur Messung des elektrischen Potentials eine Gegenelektrode in die Kochsalzlösung eingetaucht wird, dann bei Abweichung des gemessenen Potentials vom Potential des mazerierten Knochens die Mikro-/Makroporosität innerhalb der vorgegebenen Bereiche verändert wird, bis die Potentialdifferenz Null wird.

7. Verfahren nach Anspruch 7 dadurch gekennzeichnet, daß die Veränderung der Porosität durch Einwirkung von Hitze, Druck oder energiereiche Strahlung auf die Oberfläche des Implantatwerkstoffes bewirkt wird.

8. Verfahren nach Anspruch 6 oder 7 dadurch gekennzeichnet, daß das Implantatmaterial aus einer austauschbare, biokompatible Ionen enthaltenden Keramik besteht, die nach dem Plasmaspritzverfahren hergestellt wurde.

9. Verfahren nach Anspruch 8 dadurch gekennzeichnet, daß das Implantatmaterial vor dem Eintauchen in Kochsalzlösung von allen an seiner Oberfläche anhaftenden Teilchen gereinigt wurde.

## Claims

1. An osteotropic implant material containing exchangeable, biocompatible ions, the implant material comprising a microporosity with pore diameters of < 5 µm of 3-20 vol% and a macroporosity with pore widths > 100 µm of 10-60 vol%,
characterised in
that in aqueous solutions the implant material comprises a inert layer 2 (Fig. 1) of polarised calcium and phosphate ions.

2. An osteotropic implant material according to claim 1,
characterised in
that the thickness of the inert layer is 5-500 µm.

3. An osteotropic implant material according to any one of the preceding claims,
characterised in
that between the inert layer of the implant material and the 1% salt solution, there exists a potential of 10-13 mV as measured by a glass microelectrode.

4. An osteotropic implant material according to any one of the prededing claims,
characterised in
that the inept layer contains exchanged, biocompatible ions.

5. An osteotropic implant material according to any one of the preceding claims,
characterised in
that the implant material consists of hydroxyl apatite ceramics and that the inert layer comprises calcium ions.

6. A process of producing an osteotropic implant material according to any one of the preceding claims,
characterised in
that the implant material is immersed in a diluted salt solution, that after an inert layer has been formed, a glass microelectrode is introduced into the layer and that for measuring an electric potential, a counter-electrode is introduced into the salt solution and that, if the measured potential deviates from the potential of the macerated bone, the micro-/macroporosity is varied within the specified ranges until the difference in potential is zero.

7. A process according to claim 7,
characterised in
that the change in porosity is achieved by applying heat, pressure or high-energy radiation to the surface of the implant material.

8. A process according to claim 6 or 7,
characterised in
that the implant material consists of exchangeable, biocompatible, ion-containing ceramics produced in accordance with the plasma injection moulding process.

9. A process according to claim 8,
characterised in
that before the implant material is immersed in the salt solution, it is cleaned in that all particles adhering to its surface are removed.

## Revendications

1. Matériau ostéotrope pour implant contenant des ions biocompatibles, susceptibles d'un échange, le matériau de l'implant ayant une micro-porosité de 3-20 % en volume avec des diamètres de pores < 5 µm et une macro-porosité de 10-60 % en volume avec des largeurs de pores > 100 µm, caractérisé en ce que le matériau de l'implant comporte dans des solutions aqueuses une couche statique 2 (figure 1) d'ions polarisés de calcium et de phosphate.

2. Matériau ostéotrope pour implant selon la revendication 1, caractérisé en ce que l'épaisseur de la couche statique est de 5-500 µm.

3. Matériau ostéotrope pour implant selon l'une des revendications précédentes, caractérisé en ce qu'un potentiel de 10-13 mV, mesuré avec une micro-électrode de verre, règne entre la couche statique du matériau de l'implant et la solution à 1 % de chlorure de sodium.

4. Matériau ostéotrope pour implant selon l'une des revendications précédentes, caractérisé en ce que des ions biocompatibles, susceptibles d'un échange, se trouvent dans la couche statique.

5. Matériau ostéotrope pour implant selon l'une des revendications précédentes, caractérisé en ce que le matériau de l'implant est en une céramique d'hydroxylapatite et la couche statique comprend des ions calcium.

6. Procédé de fabrication d'un matériau ostéotrope pour implant selon l'une des revendications précédentes, caractérisé en ce que le matériau pour implant est immergé dans une solution diluée de chlorure de sodium, une micro-électrode de verre est introduite dans la couche statique après sa formation et une contre-électrode est immergée dans la solution de chlorure de sodium pour la mesure du potentiel électrique, puis, en cas d'écart du potentiel mesuré par rapport au potentiel de l'os ayant subi une macération, la micro/macro-porosité est modifiée dans les plages prescrites jusqu'à ce que la différence de potentiel soit nulle.

7. Procédé selon la revendication 6, caractérisé en ce que la modification de la porosité est produite par action de chaleur, pression ou rayonnement énergétique à la surface du matériau de l'implant.

8. Procédé selon la revendication 6 ou 7, caractérisé en ce que le matériau de l'implant est en une céramique contenant des ions biocompatibles susceptibles d'un échange, qui a été réalisée par un procédé de pulvérisation de plasma.

9. Procédé selon la revendication 8, caractérisé en ce qu'avant l'immersion dans la solution de chlorure de sodium, le matériau de l'implant a été nettoyé de toutes les particules adhérant à sa surface.
